# EUROPEAN PATENT SPECIFICATION

(11) **EP 4 674 470 B1**
(45) Date of publication and mention of the grant of the patent: **13.05.2026**
(21) Application number: 24185711.9
(22) Date of filing: 01.07.2024
(51) Int. Cl.: A61N 1/36, A61N 1/04

(54) **DEVICE FOR AURICULAR NERVE STIMULATION**
VORRICHTUNG ZUR AURIKULÄREN NERVENSTIMULATION
DISPOSITIF DE STIMULATION NERVEUSE AURICULAIRE

(43) Date of publication of application: 07.01.2026
(73) Proprietor: Aurimod GmbH, 1030 Wien (AT)
(72) Inventor: ZEINER, Klaus, Vienna (AT); KAMPUSCH, Stefan, Vienna (AT); MAYER, Andreas, Vienna (AT); FABIAN, Bernhard, Vienna (AT); LE, Van Hoang, Vienna (AT)
(74) Representative: SONN Patentanwälte GmbH & Co KG

(56) References cited:
- EP-B1- 2 237 831
- US-A1- 2015 238 094
- US-A1- 2024 042 203

## Description

The invention refers to a device for auricular nerve stimulation.

Publications US 2024/042203 A1, US 2015/238094 A1 and EP 2 237 831 B1 disclose devices for auricular nerve stimulation relating to the subject matter of the present invention.

Auricular nerve stimulation has gained increasing attention in recent years as a promising therapeutic approach for various medical conditions, including pain management, addiction, and mental health disorders. Several products are currently available or under development, utilizing both transcutaneous (surface electrodes) and percutaneous (needle electrodes) stimulation techniques. These devices differ in terms of electrode placement, stimulation parameters, stimulation duration, and device lifespan.

Transcutaneous stimulation typically involves the use of reusable, handheld devices that feature specialized earpieces equipped with electrodes at fixed positions on the outer ear surface. These can be clips, in-ear electrodes for the ear canal, adhesive electrodes, or some other type of surface electrodes. Reusable transcutaneous devices are often bulky and less suitable for use during normal daily life activities due to issues such as electrode contact loss during movement, which limits the stimulation time per day (often a few minutes to hours per day). These devices rely on recharging or replaceable batteries to maintain functionality.

On the other hand, percutaneous stimulators are compact units that are attached directly to the skin with adhesives. These devices utilize single-use needle electrodes for direct access to underlying nerves. The stimulator unit and fixation are usually combined in one device, which is designed for prolonged use (e.g. up to 7 days) during the patient's normal daily life. After one application, the entire device must be replaced as the power source is non-rechargeable and drained upon usage. In some embodiments the stimulator can be reused for a defined number of weeks, e.g., 2 weeks, while electrodes need to be changed weekly, and the stimulator needs to be replaced every 2 weeks, after the battery is empty.

Several existing devices include internal sensors to measure biosignals like electrocardiogram (ECG) sensors, pulse plethysmography sensors (PPG), electroencephalogram sensors (EEG), electric skin conductance sensor (ESC), electromyography sensors (EMG) and electrical field plethysmography sensors (EFPG) or inertial measurement units (IMU) to keep track of activity or posture of the patient. The information gathered with these sensors can be used to tailor the stimulation.

The current state of auricular nerve stimulation devices presents several challenges that need to be addressed to improve their usability, sustainability, and overall performance.

Reusable devices tend to be bulky, limiting the users' ability to engage in normal daily activities while using them. Additionally, the design of these devices frequently allows for only short periods of stimulation, which restricts the potential therapeutic benefits. Earpieces, which are often used for stimulation in these devices, often disengage when the patient moves and do not guarantee good electrode contact. Compliance and therapy adherence is also a significant issue with these reusable devices, as many patients find them too cumbersome to use consistently over time.

Single-use devices, while more compact, generate a significant amount of waste, raising environmental concerns and increasing costs for long-term use. In current devices, battery and electronics are often integrated into the same housing, meaning that even when the electronics could still be usable after the battery is drained, they will be disposed of together. Moreover, in some single-use devices, electrodes are fixed to the rest of the stimulator, so whenever any electrodes need to be changed, the whole device must be discarded.

Furthermore, current single-use devices tend to be highly specialized in their application due to their fixed electrode setup, which limits their potential use cases and adaptability for different medical conditions or patient needs. This inflexibility could be addressed by developing more versatile designs that allow for customizable electrode configurations, which would enable a broader range of applications and make the devices more appealing to both patients and healthcare providers.

Affordability is another challenge in the industry, as high upfront costs can make reusable devices less appealing to potential users. Meanwhile, single-use devices can be expensive for long-term use, especially considering their disposable nature.

Therefore, the instant invention aims at overcoming the drawbacks mentioned above and providing an auricular nerve stimulation device that is convenient to use, fulfills highest hygiene standards and reduces waste and costs.

In order to solve these objects, the invention provides a device for auricular nerve stimulation, comprising:
a reusable module comprising:
   - a stimulation circuit configured to generate electrical stimulation signals,
   - a controller unit configured to control a stimulation parameter of the electrical stimulation signals, such as the intensity, frequency, pulse width, and/or duration of the electrical stimulation signals,
   - a communication unit configured to enable data exchange between the controller unit and an external device, and
   - a power management unit configured to regulate and distribute electrical power to the stimulation circuit, the controller unit and the communication unit,
a plurality of single-use modules comprising:
   - a fixation module configured to be attached to a user's body,
   - a power source module,
   - an electrode module comprising at least two electrodes, and
   - optionally a sensor module,
wherein each of the single-use modules and the reusable module comprise mechanical connecting means for mechanically connecting each of the single-use modules with the reusable module in a releasable manner.

The separation of the device into a reusable module and multiple single-use modules allows for a significant reduction in waste generation compared to fully disposable devices. This modular approach maintains high hygiene standards by allowing the replacement of only those components that come into direct contact with the user, while preserving the more complex and costly electronic components for repeated use.

The modular design enables a high degree of customization and flexibility. Users can select and combine different single-use modules based on their specific therapeutic needs, resulting in a more personalized and effective treatment. For example, the electrode module can be chosen to provide either transcutaneous or percutaneous stimulation, depending on the required therapy.

The inclusion of an optional sensor module further enhances the device's adaptability. By integrating various sensor types (e.g., ECG, EEG, PPG, EMG, ECS, EFPG, IMU) into a separate module, the device can provide appropriate feedback for different applications. This feature allows for real-time monitoring and adjustment of stimulation parameters, potentially improving therapeutic outcomes.

The mechanical connecting means between the reusable module and the single-use modules ensures secure and easy connectivity while maintaining the ability to quickly exchange or replace individual components. This design contributes to improved user experience and device longevity, as it allows for the replacement of specific modules in case of wear or malfunction without necessitating the replacement of the entire device.

The modular architecture of the device results in improved cost-effectiveness. The reusable module, containing the more expensive electronic components, can be designed for long-term use, reducing the overall cost of the therapy. Simultaneously, the single-use modules can be produced more economically, as they contain fewer complex components.

The compact nature of the reusable module, combined with the flexibility of the single-use modules, allows for a device that is suitable for extended wear during normal daily activities. This represents an improvement over bulky reusable devices or limited-use disposable devices, potentially increasing patient compliance and extending the duration of effective therapy.

In the reusable module of the invention, the four components or functions - the stimulation circuit, controller unit, communication unit, and power management unit - are described as separate entities for clarity and ease of understanding. However, it is important to note that these components need not necessarily be realized as structurally separate components within the device. Instead, they can be referred to as functional definitions of specific functions that may be realized within an integrated solution.

In practice, the reusable module may be designed as a single, compact unit, where the various functions are implemented through a combination of hardware and software. For example, the controller unit and communication unit could be integrated into a single microprocessor or system-on-chip (SoC) that handles both the control of stimulation parameters and the data exchange with external devices. Similarly, the power management unit could be realized as a set of integrated circuits that work together to regulate and distribute electrical power to the other functional components.

The stimulation circuit is designed to produce electrical impulses with specific characteristics, such as waveform, frequency, pulse width, amplitude, and duration, which are suitable for stimulating the targeted auricular nerves in the region of the user's ears. In one embodiment, the stimulation circuit includes a microcontroller or processor that executes firmware or software instructions to generate the desired stimulation signals. The microcontroller is coupled to a digital-to-analog converter (DAC) that converts the digital signals generated by the microcontroller into analog waveforms. The analog waveforms are then amplified by a power amplifier to provide the necessary current and voltage levels for effective nerve stimulation.

In another embodiment, the stimulation circuit may include a waveform generator, such as a dedicated integrated circuit or a programmable logic device, that produces the desired stimulation waveforms. The waveform generator can be configured to output various waveform shapes, such as square waves, sine waves, or triangular waves, with adjustable frequency, amplitude, and duty cycle. The output of the waveform generator is then conditioned by signal conditioning circuitry, which may include filters, amplifiers, and safety limiters, before being delivered to the electrode arrangement.

The stimulation circuit is powered by the single-use power source module, which provides sufficient energy to generate the electrical stimulation signals for the duration of the intended purpose. The use of a single-use power source has several advantages over a rechargeable battery, e.g. in the reusable module, namely a higher charge capacity and thus longer possible stimulation duration and wear time without the need of intermittent recharging, limiting the necessary manipulation of the device during, e.g., seven days of continuous therapy. Taking off the device to recharge at home may limit usability and patient comfort significantly.

The controller unit may provide a user interface that allows users to easily adjust and monitor the stimulation parameters. The interface may include visual displays, such as LED indicators or a digital screen, that provide real-time information about the current stimulation settings and the device's status. Users can interact with the control unit through controls, such as buttons, dials, or touch-sensitive surfaces, to modify the stimulation parameters according to their preferences or as directed by their healthcare provider. The controller unit may also include memory functions that allow users to save and recall their preferred stimulation settings, facilitating consistent and convenient use of the device across multiple sessions. Additionally, the controller unit can be programmed to deliver pre-set stimulation protocols or therapy regimens, which can be easily selected and initiated by the user.

When stimulating the auricular nerves using transcutaneous or percutaneous electrical stimulation, several key parameters are considered and adjusted to optimize the therapy. These parameters include the intensity (amplitude), frequency, pulse width, and duration of the stimulation signals. The selection and adjustment of these parameters depend on various factors, such as the targeted nerve branches, the desired physiological effects, and the individual user's tolerance and response to the stimulation.

### Intensity (Amplitude):

The intensity or amplitude of the stimulation refers to the strength of the electrical current delivered to the auricular nerves. It is typically measured in milliamperes (mA) and is adjusted to ensure that the stimulation is perceptible but not uncomfortable for the user. The optimal intensity level varies among individuals and is determined through a gradual ramping-up process, starting from a low intensity and increasing until the user feels a tingling or mild buzzing sensation. The intensity is usually set at a level just below the user's discomfort threshold to maximize the therapeutic effects while minimizing any unpleasant sensations. Typical intensity ranges for auricular nerve stimulation are between 0.5 mA to 5 mA, depending on the user's sensitivity and the electrode placement. In another embodiment, especially when using needle electrodes for percutaneous stimulation, constant voltage stimulation may be utilized, allowing the adjustment of the voltage of the stimulation waveform while the current is determined by the impedance of the electrode-tissue interface. Typical intensity ranges for auricular nerve stimulation are between 0.5 V to 5 V, depending on the user's sensitivity and the electrode placement.

### Frequency:

The frequency of the stimulation refers to the number of electrical pulses delivered per second, measured in Hertz (Hz). The choice of stimulation frequency is based on the desired physiological effects and the specific nerve fibers being targeted.

### Pulse Width:

The pulse width refers to the duration of each electrical pulse, measured in microseconds (µs). It determines the amount of time the stimulation current is applied to the nerves during each pulse. Longer pulse widths can recruit more nerve fibers and produce stronger physiological responses, but they may also increase the likelihood of discomfort. Shorter pulse widths are generally more comfortable but may require higher intensities to achieve the desired effects. Typical pulse widths used in auricular nerve stimulation range from 50 µs to 500 µs, with 200-300 µs being commonly employed.

### Duration:

The duration of the stimulation refers to the length of each stimulation session or the overall treatment period. The optimal duration depends on the specific therapeutic goals and the user's response to the stimulation. Shorter sessions (10-60 minutes), sometimes repeated 3-4 times over one day, are often used for acute symptom relief, while longer sessions (30-120 minutes), often repeated continuously only with short pauses (30-60 minutes) in between stimulation sessions, may be employed for chronic conditions. The duration of the stimulation can be gradually decreased (weaning off) or increased over time to maintain the therapeutic effects, to prevent adaptation or habituation to the stimulation, or to account for a long-lasting therapeutic effect which builds-up over prolonged periods of stimulation (2-6 weeks or longer) and thus reduces the need for further stimulation therapy.

In order to allow the controller unit to exchange data with external devices, the reusable module comprises a communication unit. This function enables the device to receive instructions, transmit data, and synchronize with other systems.

In one embodiment, the communication unit is provided to allow the controller unit to receive stimulation parameters, such as intensity, frequency, and duration, from external devices. This enables healthcare providers to customize and adjust the therapy remotely, based on the patient's needs and progress. Additionally, the communication unit can transmit data collected by the device, such as sensor readings or device usage information, to external systems for monitoring and analysis. This data can be used to track patient compliance, assess treatment efficacy, and make informed decisions about treatment modifications.

There are several possible technical embodiments of the communication unit, depending on the specific requirements of the device and its intended purpose. In one embodiment, wireless communication protocols are used, such as Bluetooth Low Energy (BLE), Wi-Fi, or Near Field Communication (NFC). These protocols allow for secure, short-range data transmission between the device and external systems, such as smartphones, tablets, or dedicated medical devices. The communication unit could be implemented using a dedicated wireless module or integrated into a microcontroller or SoC that also handles other functions of the reusable module.

Another possible embodiment is to use wired communication interfaces, such as USB, for data exchange. This embodiment may be suitable for devices that require high-speed data transfer or need to be physically connected to external systems for programming or readout of sensor data to provide real-time feedback for the control unit and stimulation circuit. In this case, the communication unit would include the necessary hardware components, such as connectors and interface circuits, to enable reliable wired communication.

The power management unit in the reusable module is responsible for regulating and distributing electrical power to the stimulation circuit, controller unit, communication unit, and optional sensor units. Its primary function is to ensure that each component receives the appropriate voltage and current levels required for optimal performance while minimizing power consumption and extending battery life.

The fixation module, one of the single-use modules of the invention, serves the function of securely attaching the device to the user's body. The fixation module is designed to be mechanically connected to the reusable module in a releasable manner, allowing for easy replacement of the single-use components while preserving the integrity of the reusable module.

The mechanical connection between the fixation module and the reusable module can be achieved through various means, such as snap-fit connectors, magnets, or friction-fit mechanisms.

On the user-facing side, the fixation module is configured to be attached to the user's body. Several attachment methods can be employed, depending on the specific application and user preferences. In a preferred embodiment, the fixation module is designed to be adhesively attached to the user's body. This adhesive attachment can be achieved through the use of an adhesive patch that is integrated into the fixation module. The adhesive patch may comprise a release liner that is removed prior to application, exposing the adhesive surface. Alternative attachment methods for the fixation module may include the use of straps, ear clips, or headbands. These embodiments can be employed in situations where adhesive attachment is not preferred or feasible, such as for users with sensitive skin or in applications that require frequent device repositioning.

The electrode module, another single-use module of the invention, is responsible for delivering the electrical stimulation signals generated by the stimulation circuit to the targeted neural structures. This module comprises at least two electrodes, which serve as the interface between the device and the user's body. The electrode module can be implemented using various electrode types and configurations, depending on the specific stimulation modality and target site. In a preferred embodiment, the electrode module comprises needle electrodes for delivering percutaneous stimulation. Needle electrodes are thin, conductive needles that can penetrate the skin and directly contact the underlying neural structures. This embodiment allows for highly localized and precise stimulation, as the needle electrodes can be positioned in close proximity to the targeted nerves. Needle electrodes can be made of biocompatible materials, such as titanium, stainless steel or platinum-iridium alloys. The length and diameter of the needle electrodes can be optimized based on the intended application site and the desired depth of penetration.

Alternatively, the electrode module may comprise surface electrodes for delivering transcutaneous stimulation. Surface electrodes are non-invasive electrodes that are placed on the skin surface over the targeted neural structures. These electrodes can be made of conductive materials, such as hydrogel, conductive plastics, or metallic bodies and foils, and can be designed in various shapes and sizes to accommodate different anatomical regions. Surface electrodes can be coupled to the skin using conductive adhesives or mechanical pressure.

Regardless of the electrode type, the electrode module is designed to be easily and securely connected to the reusable module, allowing for a modular and replaceable single-use component. This connection can be achieved through the use of standard connector interfaces, such as snap connectors or pin headers, which provide a reliable electrical and mechanical coupling between the electrode module and the reusable module taking into account also multiple mating cycles of the reusable module.

In a preferred embodiment, the electrodes of the electrode module can be connected to the single-use electrode module or directly to the reusable module using electrical cables. These cables feature connectors that serve as the connecting means for establishing an electrical and mechanical connection with the mating connecting means located on the housing of the single-use module or the reusable module, respectively. The use of electrical cables allows for greater flexibility in electrode placement, as the electrodes can be positioned independently of the reusable module. This can be particularly advantageous in situations where the optimal stimulation site is not in close proximity to the reusable module.

Preferably, the controller unit may be configured to detect which type of electrode module is connected to the reusable module, and optionally adjust at least one stimulation parameter of the electrical stimulation signals based on the type of electrode module.

The auto-detect feature, which enables the controller unit to automatically identify which type of electrode module is connected to the reusable module, can be implemented through various embodiments. One possible embodiment of the auto-detect feature involves the use of unique identification resistors or ID chips within each electrode module. In this approach, the electrode modules are equipped with resistors of different predetermined values or ID chips with unique digital codes. When an electrode module is connected to the reusable module, the controller unit measures the resistance value or reads the digital code through the connection means. By comparing the obtained value or code with a predefined lookup table or database, the controller unit can determine which specific type of electrode module is currently connected. This allows the controller unit to configure its stimulation parameter adjustments based on the identified electrode type, such as transcutaneous or percutaneous electrodes.

The power source module is responsible for supplying electrical energy to the reusable module and its associated functional units. In one embodiment, the power source module is implemented in a housing separate from the housing of the reusable module. The separate housing of the power source module can be designed to be mechanically and electrically connected to the reusable module through the use of appropriate connecting means, such as snap-fit connectors or sliding contacts. This connection should ensure a secure and reliable power transfer while also allowing for easy detachment and replacement of the power source module when necessary.

Alternatively, the power source module can be integrated into the reusable module itself. In this embodiment, the reusable module's housing includes a dedicated compartment that is designed to receive and secure the power source module. The compartment can be accessed through a removable cover or door, allowing for easy insertion and removal of the power source module.

The power source module can be implemented using various battery technologies. Commonly used battery types include disposable alkaline batteries, rechargeable lithium-ion or lithium-polymer batteries, or coin cell batteries. In addition to the battery itself, the power source module may incorporate additional features, such as battery level indicators, protection circuits, or charging interfaces.

In addition to the mechanical connection of the single-use modules to the reusable module, a preferred embodiment provides that the reusable module, the electrode module, the power source module and the optional sensor module comprise electrical connecting means for electrically connecting the electrode module, the power source module and the optional sensor module, respectively, with the reusable module in a releasable manner. The electrical connecting means can be implemented using various connector types and configurations. Preferably, the mechanical connection means and the electrical connection means are formed integrally, resulting in that the electrical connection is established in conjunction with the mechanical connection.

Generally speaking, the mechanical connecting means are configured to establish at least one of a magnetic connection, a friction-fit connection and a form-fit connection.

In one embodiment, the connection means comprise snap-fit connectors, which provide a reliable and intuitive connection method. In this embodiment, the reusable module may comprise male snap-fit connectors that protrude from its housing, while the electrode module, power source module, and sensor module may comprise corresponding female snap-fit connectors. When the modules are brought together, the male and female connectors engage, establishing an electrical and mechanical connection between the respective modules. The snap-fit design ensures proper alignment and orientation of the connectors, preventing accidental mismatch or short-circuits. Additionally, the snap-fit mechanism provides a tactile and audible feedback to the user, confirming a successful connection.

Alternatively, the electrical connecting means can be realized through the use of magnetically coupled connectors. In this embodiment, the reusable module includes magnetic contacts or pins that are exposed on its housing. Correspondingly, the electrode module, power source module, and sensor module comprise mating magnetic contacts or receptacles that align with the contacts on the reusable module. When the modules are brought into close proximity, the magnetic attraction between the contacts ensures a secure and reliable mechanical and electrical connection. This magnetic coupling approach offers the advantage of a self-aligning and self-locating connection, which enhances user convenience and reduces the risk of misalignment.

According to a preferred embodiment of the invention, at least two of the single-use modules are combined in one housing, the combination being selected from the group consisting of:
a) the power source module and the electrode module,
b) the electrode module and the fixation module,
c) the power source module and the fixation module,
d) the power source module, the electrode module, and the fixation module,
e) the sensor module and the power source module, and
f) the power source module, the electrode module, and the sensor module and optionally the fixation module.

One possible combination is the integration of the power source module and the electrode module into a single housing. In this configuration, the power source, such as a disposable battery, is housed within the same enclosure as the stimulation electrodes or electrode cable connection. This embodiment provides a compact and self-contained stimulation unit that can be easily attached to and detached from the reusable module. The integrated housing can be designed with appropriate electrical connecting means, such as snap-fit connectors or magnetically coupled contacts, to establish a secure and reliable electrical connection with the reusable module. By combining the power source and electrodes into a single unit, the user can replace both components simultaneously, simplifying the maintenance and operation of the device.

Another advantageous combination is the integration of the electrode module and the fixation module into a single housing. In this embodiment, the stimulation electrodes are incorporated into the same structure that is responsible for attaching the device to the user's body. This integration can be beneficial for applications where a direct and stable contact between the electrodes and the skin is crucial. The combined electrode-fixation module can be designed with an adhesive layer on the skin-facing side, allowing for a secure and conformable attachment to the user's body.

A third possibility is the integration of the power source module and the fixation module into a single housing. The power source, such as a coin cell battery or a thin-film battery, can be embedded within the fixation module's housing.

Lastly, an integrated embodiment combines the power source module, electrode module, and fixation module into a single unit. This design offers a highly compact and convenient solution, where all the single-use components are housed within a single, replaceable module. The power source, stimulation electrodes, and fixation mechanism are all integrated into a common housing, which can be easily attached to and detached from the reusable module. This embodiment minimizes the number of separate components and simplifies handling of the device. The integrated module can be designed with appropriate electrical and mechanical connecting means to ensure a secure and reliable interface with the reusable module.

In a preferred embodiment of the invention, the plurality of single-use modules includes a sensor module, which is designed to collect physiological and/or environmental data relevant to the stimulation therapy or the user's condition. The sensor module is integrated into the modular architecture of the device, allowing for the easy replacement and customization of the sensing capabilities based on the specific application requirements.

Alternatively, the sensor module may also be designed as a reusable module or be integrated into the reusable module.

The sensor module preferably comprises at least one sensor selected from the group consisting of an electrocardiogram (ECG) sensor, an electroencephalogram (EEG) sensor, a photoplethysmography (PPG) sensor, an electromyography sensor (EMG), an electric skin conductance (ESC) sensor, an electromyography (EMG) sensor, an electrical field plethysmography (EFPG) sensor and an inertial measurement unit (IMU) sensor. Each of these sensors provides information that can be used to monitor the user's physiological state, assess the effectiveness of the stimulation therapy, or adapt the stimulation parameters in real-time.

The ECG sensor is designed to measure the electrical activity of the user's heart, providing insights into the cardiac function and potential abnormalities. The ECG sensor typically comprises a set of electrodes that are placed on the user's skin, either directly or through the fixation module, to detect the electrical signals generated by the heart. The sensor module can include the necessary circuitry for signal conditioning, amplification, and digitization, as well as algorithms for heart rate detection and arrhythmia analysis. Alternatively, said circuitry and algorithms may be provided in the controller unit. The ECG data can be used to monitor the user's cardiac response to the stimulation therapy including changes in heart rate variability used to detect changes in autonomic nervous system function, detect any adverse effects, or trigger stimulation based on specific cardiac events.

The EEG sensor is designed to measure the electrical activity of the user's brain, providing valuable information about the neural state and potential abnormalities. The EEG sensor typically comprises a set of electrodes that are placed on the user's scalp, either directly or through a separate fixation mechanism, to detect the electrical signals generated by the brain. The sensor module can include the necessary circuitry for signal conditioning, amplification, and digitization, as well as algorithms for spectral analysis, event detection, and artifact removal. Alternatively, said circuitry and algorithms may be provided in the controller unit. The EEG data can be used to monitor the user's neural response to the stimulation therapy, assess the level of arousal or relaxation, or trigger stimulation based on specific brain wave patterns.

The PPG sensor is designed to measure the user's blood volume changes, providing information about the cardiovascular function and potential abnormalities. The PPG sensor typically comprises a light source, such as a LED, and a photodetector, which are placed in close proximity to the user's skin, such as the earlobe, or other suitable location, e.g., below the fixation module on the sternocleidomastoid muscle on the neck. The sensor module can include the necessary circuitry for light emission, detection, and signal processing, as well as algorithms for heart rate extraction, blood oxygen saturation estimation, and respiratory rate monitoring. Alternatively, said circuitry and algorithms may be provided in the controller unit. The PPG data can be used to monitor the user's cardiovascular response to the stimulation therapy, detect any adverse effects, or trigger stimulation based on specific physiological parameters.

The EMG sensor is designed to measure the electrical activity of specific muscle groups of the user, providing insights into the muscle tone and contraction pattern of muscles and potential abnormalities. The EMG sensor typically comprises a set of electrodes that are placed on the user's skin, either directly or through the fixation module, to detect the electrical signals generated by the muscle contraction. The sensor module can include the necessary circuitry for signal conditioning, amplification, and digitization, as well as algorithms for twitch detection an analysis of the muscle tone. Alternatively, said circuitry and algorithms may be provided in the controller unit. The EMG data can be used to monitor the user's muscle tone and contraction changes in response to the stimulation therapy including changes in contraction patterns, state of muscle relaxation, or trigger stimulation based on specific myographic events.

The EFPG sensor is designed to measure the user's blood or air volume changes, providing information about the cardiovascular and respiratory function and potential abnormalities. The EFPG sensor typically comprises a current source and a voltage measurement circuit to determine changes in tissue impedance and more than three electrodes, which are placed in close proximity to the user's skin, such as the chest, or other suitable locations, e.g., below the fixation module on the sternocleidomastoid muscle on the neck. Tissue impedance changes due to changes in blood volume or air volume in specific body regions due to respiration and cardiac activity. The sensor module can include the necessary circuitry for current generation, voltage detection, and signal processing, as well as algorithms for heart rate extraction and respiratory rate monitoring. Alternatively, said circuitry and algorithms may be provided in the controller unit. The EFPG data can be used to monitor the user's cardiovascular and respiratory response to the stimulation therapy, detect any adverse effects, or trigger stimulation based on specific physiological parameters.

The ESC or galvanic skin resistance sensor is designed to measure the user's change in skin conductance due to perspiration, providing information on autonomic function and stress level of the patient. The ESC sensor typically comprises a current source and measures changes in skin resistance via electrodes, which are placed in close proximity to the user's skin, e.g., below the fixation module on the sternocleidomastoid muscle on the neck. The sensor module can include the necessary circuitry for current generation, voltage detection, and signal processing, as well as algorithms for detection of changes in stress level and autonomic function. Alternatively, said circuitry and algorithms may be provided in the controller unit. The ESC data can be used to monitor the user's autonomic function response to the stimulation therapy, detect any adverse effects, or trigger stimulation based on specific physiological parameters.

The IMU sensor is designed to measure the user's motion and orientation, providing information about the physical activity and posture. The IMU sensor typically comprises a combination of accelerometers, gyroscopes, and magnetometers, which are integrated into the sensor module. The sensor can detect linear acceleration, angular velocity, and magnetic field changes, allowing for the tracking of the user's movements and orientation in three-dimensional space. The IMU data can be used to monitor the user's physical activity levels, or other abnormal movements, or adapt the stimulation parameters based on the user's posture or motion. An IMU sensor may also be used to detect specific muscle contractions, cardiac or respiration events depending on the position of the sensor on the body of the patient.

The sensor module can be designed as a separate single-use or multiple-use component that is mechanically and electrically connected to the reusable module, or it can be integrated into one of the other single-use modules or the reusable module, such as the electrode module or the fixation module. The sensor module can include the necessary electrical interfaces, such as connectors or flexible circuits, to establish a reliable and secure connection with the reusable module. The sensor data can be transmitted to the reusable module's controller unit for further processing, storage, or transmission to external devices via the communication unit.

In a preferred embodiment of the invention, the power source module is designed to provide electrical power to the reusable module's power management unit when the two modules are electrically connected. This electrical connection is established through the use of appropriate electrical connecting means, such as connectors or contacts, which are present on both the power source module and the reusable module.

The power source module typically comprises one or more batteries, which can be disposable. When the power source module is electrically connected to the reusable module, the batteries within the power source module provide a DC voltage to the power management unit of the reusable module. The power management unit is responsible for regulating and distributing the power from the batteries to the various components of the reusable module, such as the controller unit, communication unit, and stimulation unit.

In addition to voltage regulation, the power management unit may also include battery monitoring and protection circuitry. This circuitry can monitor the battery voltage, current, and temperature to ensure safe and efficient operation. If the battery voltage drops below a certain threshold, the monitoring circuitry can alert the controller unit, which may then notify the user or take appropriate action. The power management unit may also include power-saving features, such as sleep modes or low-power modes, which can be activated when the device is not in use or when certain components are not required. These power-saving features help to extend the battery life and reduce the frequency of battery replacements.

In a preferred embodiment of the invention, the sensor module, when electrically connected to the reusable module, receives electrical power from the power management unit. When the sensor module is electrically connected to the reusable module, the power management unit directs the electrical power from the power source module to the sensor module. By utilizing the power from the power source module, the sensor module can operate without the need for its own separate battery or power supply. This not only simplifies the design of the sensor module but also reduces the overall size and weight of the device, as well as the cost and complexity associated with having multiple power sources.

In a preferred embodiment of the invention, the electrical connecting means of the reusable module for connecting the sensor module to the reusable module are configured to transmit sensor data from the sensor module to the controller unit. In one embodiment, the electrical connecting means may include a dedicated data bus or communication interface for the transmission of sensor data. These interfaces provide a standardized protocol for data exchange, allowing the sensor module to send digital sensor data to the controller unit in a structured manner. The data bus may be implemented using a subset of the connector pins or contacts, which are allocated specifically for data transmission.

In addition to transmitting sensor data, the electrical connecting means may also support bidirectional communication between the sensor module and the reusable module. This allows the controller unit to send configuration commands, firmware updates, or other instructions to the sensor module, enabling adjustments of the sensor parameters or modes of operation. Bidirectional communication can also facilitate the implementation of advanced features, such as sensor self-calibration or self-test routines, which can enhance the accuracy and reliability of the sensor measurements.

In a preferred embodiment of the invention, the controller unit is configured to receive the sensor data from the sensor module, process the received sensor data, and adjust at least one stimulation parameter of the electrical stimulation signals based on the processed sensor data. This embodiment enables the device to adapt the stimulation therapy in real-time, based on the user's physiological or environmental conditions, thereby enhancing the efficacy of the treatment. Upon receiving the sensor data, the controller unit may process the data using various signal processing algorithms and techniques. These may include filtering, amplification, noise reduction, feature extraction, and pattern recognition, among others. For example, if the sensor module includes an ECG sensor, the controller unit may process the ECG signals to extract heart rate, heart rate variability, or other cardiac parameters.

Based on the processed sensor data, the controller unit is configured to adjust at least one stimulation parameter of the electrical stimulation signals delivered by the stimulation unit. The stimulation parameters that can be adjusted may include, but are not limited to, pulse amplitude, pulse width, pulse frequency, duty cycle, or stimulation waveform.

The controller unit may utilize various control algorithms, such as proportional-integral-derivative (PID) control, fuzzy logic control, or machine learning techniques, to determine the optimal adjustments to the stimulation parameters based on the processed sensor data. These algorithms can take into account multiple input variables, historical data, and therapeutic goals to make intelligent and adaptive decisions regarding the stimulation therapy.

In addition to or instead of adjusting the stimulation parameters, the controller unit may also use the processed sensor data to trigger specific stimulation programs or modes, or to provide feedback to the user or healthcare provider regarding the progress or effectiveness of the therapy. The controller unit may store the processed sensor data and the corresponding stimulation parameters in the memory unit of the reusable module for later analysis or review, such as by transmitting the sensor data to an external device via the communication unit.

According to another preferred embodiment of the invention, the reusable module may comprise a signal acquisition circuit, preferably for EFPG measurements, electrically connected to the electrical connecting means for connecting the electrode module to the reusable module, the signal acquisition circuit being configured to measure and record electrical impedance and potential signals from the tissue underlying the electrodes, and a signal processing unit electrically connected to the signal acquisition circuit, the signal processing unit being configured to analyze the measured electrical impedance and potential signals to extract physiological information, such as heart rate, heart rate variability, and respiration rate. By employing the same electrodes for both stimulation and sensing, the device can achieve a more compact and integrated design, as it eliminates the need for separate sets of electrodes dedicated to each function. This integration simplifies the overall system architecture, and reduces the number of components. EFPG measurements may be carried out as disclosed in EP 4252829 A1.

Preferably, the controller unit may be configured to receive the physiological information from the signal processing unit and use the physiological information to adapt the stimulation parameters of the electrical stimulation circuit based on the user's current physiological state and/or response to the electrical stimulation.

According to a preferred embodiment, the inventive device comprises at least two sensor modules that have different types of sensors.

Preferably, a first sensor module of the at least two sensor modules comprises one sensor selected from the group consisting of an electrocardiogram sensor, an electroencephalogram sensor, a photoplethysmography sensor, an electromyography sensor, an electrical field plethysmography sensor, an electrical skin conductance sensor, and an inertial measurement unit sensor, and a second sensor module of the at least two sensor modules comprises another sensor selected from the group consisting of an electrocardiogram sensor, an electroencephalogram sensor, a photoplethysmography sensor, an electromyography sensor, an electrical field plethysmography sensor, an electrical skin conductance sensor, and an inertial measurement unit sensor.

The modular aspect of the device, as addressed in the second aspect of the invention, enhances the versatility, adaptability, and user-friendliness of the auricular nerve stimulation system. By offering at least two sensor modules with different types of sensors, the modular design allows users to customize the device according to their specific monitoring needs and preferences. This modularity enables a single reusable module to be combined with various sensor modules, each equipped with different sensors such as an electrocardiogram sensor, an electroencephalogram sensor, a photoplethysmography sensor, an electromyography sensor, an electrical field plethysmography sensor, an electrical skin conductance sensor, or an inertial measurement unit sensor. Users can easily swap or interchange these sensor modules, depending on the physiological parameters they wish to monitor or the specific therapeutic applications they require. This flexibility in sensor selection empowers users to tailor the device to their individual needs, ensuring a more personalized and targeted approach to auricular nerve stimulation. Moreover, the modular design streamlines the manufacturing process, as different sensor modules can be produced independently and combined with the reusable module as needed, reducing production costs and increasing the overall efficiency of the system. The modular aspect also simplifies maintenance and repair procedures, as individual sensor modules can be replaced or upgraded without the need to replace the entire device, thereby extending the lifespan of the system and reducing long-term costs for the user.

According to a preferred embodiment of the invention, the first sensor module and the second sensor module each comprise a connecting means configured to mate with the connection means of the reusable module, and the connection means of the reusable module is configured to receive and establish an electrical and mechanical connection with either the first sensor module or the second sensor module, and wherein the controller unit is configured to detect which of the first sensor module or the second sensor module is connected to the connection means of the sensor module and to receive and process data from the connected sensor module, and optionally adjust at least one stimulation parameter of the electrical stimulation signals based on the processed data from the connected sensor module.

By incorporating a single connection means on the reusable module that is configured to mate with the connecting means of either the first sensor module or the second sensor module, the device eliminates the need for multiple interfaces. Moreover, the controller unit's ability to detect which sensor module is connected and automatically adapt its data processing and stimulation parameter adjustment accordingly further enhances the device's ease of use.

The auto-detect feature, which enables the controller unit to automatically identify which sensor module is connected to the reusable module, can be implemented through various embodiments. These embodiments rely on different techniques to distinguish between the first and second sensor modules, ensuring that the controller unit can process the received data correctly and adjust the stimulation parameters accordingly.

One possible embodiment of the auto-detect feature involves the use of unique identification resistors or ID chips within each sensor module. In this approach, the first and second sensor modules are equipped with resistors of different predetermined values or ID chips with unique digital codes. When a sensor module is connected to the reusable module, the controller unit measures the resistance value or reads the digital code through the connection means. By comparing the obtained value or code with a predefined lookup table or database, the controller unit can determine which specific sensor module is currently connected. This allows the controller unit to identify the type of sensors present in the connected module and configure its data processing algorithms and stimulation parameter adjustments based on the identified sensor type.

In the following, the invention will be described with reference to an exemplary embodiment illustrated in the drawing.

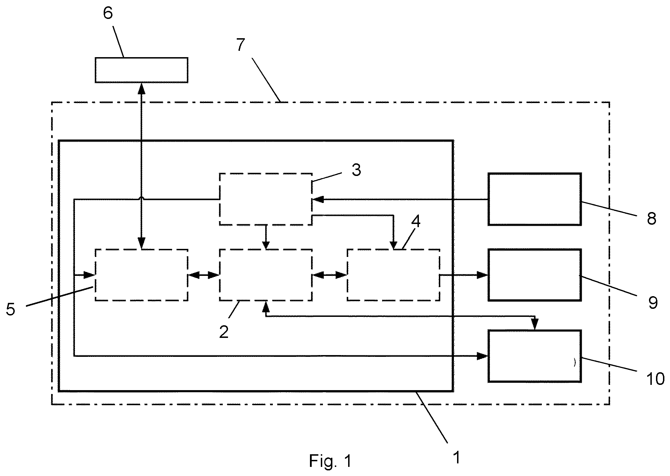

Fig. 1 shows a block diagram of a device for auricular nerve stimulation. The stimulation device comprises a reusable module 1, which comprises a controller unit 2, a power management unit 3, a stimulation circuit 4 and a communication unit 5. The communication unit 5 is configured to exchange data with a remote control 6 wirelessly or via a cable. Further, the device comprises a plurality of single-use modules, i.e. a fixation module 7 configured to be attached to a user's body, a power source module 8, an electrode module 9 and a sensor module 10.

## Claims

1. A device for auricular nerve stimulation, comprising:
a reusable module (1) comprising:
- a stimulation circuit (4) configured to generate electrical stimulation signals,
- a controller unit (2) configured to control a stimulation parameter of the electrical stimulation signals, such as the intensity, frequency, pulse width, and/or duration of the electrical stimulation signals,
- a communication unit (5) configured to enable data exchange between the controller unit (2) and an external device (6), and
- a power management unit (3) configured to regulate and distribute electrical power to the stimulation circuit (4), the controller unit (2) and the communication unit(5),
a plurality of single-use modules comprising:
- a fixation module (7) configured to be attached to a user's body,
- a power source module (8),
- an electrode module (9) comprising at least two electrodes, and
- optionally a sensor module (10),
wherein each of the single-use modules (7,8,9,10) and the reusable module (1) comprise mechanical connecting means for mechanically connecting each of the single-use modules (7,8,9,10) with the reusable module (1) in a releasable manner.

2. Device according to claim 1, wherein the reusable module (1), the electrode module (9), the power source module (8) and the optional sensor (10) module comprise electrical connecting means for electrically connecting the electrode module (9), the power source module (8) and the optional sensor module (10), respectively, with the reusable module (1) in a releasable manner.

3. Device according to claim 1 or 2, wherein the mechanical connecting means are configured to establish at least one of a magnetic connection, a friction-fit connection and a form-fit connection.

4. Device according to claim 1, 2 or 3, wherein the stimulation circuit (4), the controller unit (2), the communication unit (5) and the power management unit (3) are arranged within a single housing of the reusable module (1).

5. Device according to any one of claims 1 to 4, wherein the electrode module (9) comprises needle electrodes for delivering percutaneous stimulation or surface electrodes for delivering transcutaneous stimulation.

6. Device according to any one of claims 1 to 5, wherein the fixation module (7) is configured to be adhesively attached to a user's body.

7. Device according to any one of claims 1 to 6, wherein at least two of the single-use modules are combined in one housing, the combination being selected from the group consisting of:
a) the power source module (8) and the electrode module (9),
b) the electrode module (9) and the fixation module (7),
c) the power source module (8) and the fixation module (7),
d) the power source module (8), the electrode module (9), and the fixation module (7),
e) the sensor module (10) and the power source module (8), and
f) the power source module (8), the electrode module (9), and the sensor module (10) and optionally the fixation module (7).

8. Device according to any one of claims 1 to 7, wherein the sensor module (10) comprises at least one sensor selected from the group consisting of an electrocardiogram sensor, an electroencephalogram sensor, a photoplethysmography sensor, and electromyography sensor, an electrical field plethysmography sensor, an electric skin conductance sensor, and an inertial measurement unit sensor.

9. Device according to any one of claims 1 to 8, wherein the power source module (8), when electrically connected to the reusable module (1), provides electrical power to the power management unit (3).

10. Device according to any one of claims 1 to 9, wherein the sensor module (10), when electrically connected to the reusable module (1), receives electrical power from the power management unit (3).

11. Device according to any one of claims 2 to 10, wherein the electrical connecting means of the reusable module (1) for connecting the sensor module (10) to the reusable module (1) are configured to transmit sensor data from the sensor module (10) to the controller unit (2).

12. Device according to any one of claims 1 to 11, wherein the controller unit (2) is configured to receive the sensor data from the sensor module (10), process the received sensor data, and adjust at least one stimulation parameter of the electrical stimulation signals based on the processed sensor data.

13. Device according to any one of claims 1 to 12, wherein the reusable module (1) comprises a signal acquisition circuit electrically connected to the electrical connecting means for connecting the electrode module (9) to the reusable module (1), the signal acquisition circuit being configured to measure and record electrical impedance and potential signals from the tissue underlying the electrodes, and a signal processing unit electrically connected to the signal acquisition circuit, the signal processing unit being configured to analyze the measured electrical impedance and potential signals to extract physiological information, such as heart rate, heart rate variability, and respiration rate.

14. Device according to claim 13, wherein the controller unit (2) is configured to receive the physiological information from the signal processing unit and use the physiological information to adapt the stimulation parameters of the electrical stimulation circuit (4) based on the user's current physiological state and/or response to the electrical stimulation.

15. Device according to any one of claims 1 to 14 comprising at least two sensor modules (10) that have different types of sensors.

16. Device according to claim 15, wherein a first sensor module (10) of the at least two sensor modules comprises one sensor selected from the group consisting of an electrocardiogram sensor, an electroencephalogram sensor, a photoplethysmography sensor, an electromyography sensor, an electric skin conductance sensor, an electrical field plethysmography sensor, and an inertial measurement unit sensor, and a second sensor module (10) of the at least two sensor modules comprises another sensor selected from the group consisting of an electrocardiogram sensor, an electroencephalogram sensor, a photoplethysmography sensor, an electromyography sensor, an electric skin conductance sensor, an electrical field plethysmography sensor, and an inertial measurement unit sensor.

17. Device according to claim 16, wherein the first sensor module (10) and the second sensor module (10) each comprise a connecting means configured to mate with the connection means of the reusable module, and the connection means of the reusable module is configured to receive and establish an electrical and mechanical connection with either the first sensor module or the second sensor module, and wherein the controller unit is configured to detect which of the first sensor module or the second sensor module is connected to the connection means of the sensor module and to receive and process data from the connected sensor module, and optionally adjust at least one stimulation parameter of the electrical stimulation signals based on the processed data from the connected sensor module.

## Patentansprüche

1. Eine Vorrichtung zur aurikulären Nervenstimulation, umfassend:
ein wiederverwendbares Modul (1), umfassend:
- eine Stimulationsschaltung (4), die konfiguriert ist, um elektrische Stimulationssignale zu erzeugen,
- eine Steuereinheit (2), die konfiguriert ist, um einen Stimulationsparameter der elektrischen Stimulationssignale, wie die Intensität, Frequenz, Pulsbreite und/oder Dauer der elektrischen Stimulationssignale, zu steuern,
- eine Kommunikationseinheit (5), die konfiguriert ist, um einen Datenaustausch zwischen der Steuereinheit (2) und einer externen Vorrichtung (6) zu ermöglichen, und
- eine Energieverwaltungseinheit (3), die konfiguriert ist, um elektrische Energie zu regeln und an die Stimulationsschaltung (4), die Steuereinheit (2) und die Kommunikationseinheit (5) zu verteilen,
eine Vielzahl von Einwegmodulen, umfassend:
- ein Fixierungsmodul (7), das konfiguriert ist, um am Körper eines Benutzers angebracht zu werden,
- ein Energiequellenmodul (8),
- ein Elektrodenmodul (9), das mindestens zwei Elektroden umfasst, und
- optional ein Sensormodul (10),
wobei jedes der Einwegmodule (7, 8, 9, 10) und das wiederverwendbare Modul (1) mechanische Verbindungsmittel zum mechanischen Verbinden jedes der Einwegmodule (7, 8, 9, 10) mit dem wiederverwendbaren Modul (1) auf lösbare Weise umfassen.

2. Vorrichtung nach Anspruch 1, wobei das wiederverwendbare Modul (1), das Elektrodenmodul (9), das Energiequellenmodul (8) und das optionale Sensormodul (10) elektrische Verbindungsmittel zum elektrischen Verbinden des Elektrodenmoduls (9), des Energiequellenmoduls (8) bzw. des optionalen Sensormoduls (10) mit dem wiederverwendbaren Modul (1) auf lösbare Weise umfassen.

3. Vorrichtung nach Anspruch 1 oder 2, wobei die mechanischen Verbindungsmittel konfiguriert sind, um mindestens eine von einer magnetischen Verbindung, einer kraftschlüssigen Verbindung und einer formschlüssigen Verbindung herzustellen.

4. Vorrichtung nach Anspruch 1, 2 oder 3, wobei die Stimulationsschaltung (4), die Steuereinheit (2), die Kommunikationseinheit (5) und die Energieverwaltungseinheit (3) innerhalb eines einzigen Gehäuses des wiederverwendbaren Moduls (1) angeordnet sind.

5. Vorrichtung nach einem der Ansprüche 1 bis 4, wobei das Elektrodenmodul (9) Nadelelektroden zur Abgabe einer perkutanen Stimulation oder Oberflächenelektroden zur Abgabe einer transkutanen Stimulation umfasst.

6. Vorrichtung nach einem der Ansprüche 1 bis 5, wobei das Fixierungsmodul (7) konfiguriert ist, um klebend am Körper eines Benutzers angebracht zu werden.

7. Vorrichtung nach einem der Ansprüche 1 bis 6, wobei mindestens zwei der Einwegmodule in einem Gehäuse kombiniert sind, wobei die Kombination ausgewählt ist aus der Gruppe bestehend aus:
a) dem Energiequellenmodul (8) und dem Elektrodenmodul (9),
b) dem Elektrodenmodul (9) und dem Fixierungsmodul (7),
c) dem Energiequellenmodul (8) und dem Fixierungsmodul (7),
d) dem Energiequellenmodul (8), dem Elektrodenmodul (9) und dem Fixierungsmodul (7),
e) dem Sensormodul (10) und dem Energiequellenmodul (8), und
f) dem Energiequellenmodul (8), dem Elektrodenmodul (9) und dem Sensormodul (10) und optional dem Fixierungsmodul (7) .

8. Vorrichtung nach einem der Ansprüche 1 bis 7, wobei das Sensormodul (10) mindestens einen Sensor umfasst, der ausgewählt ist aus der Gruppe bestehend aus einem Elektrokardiogrammsensor, einem Elektroenzephalogrammsensor, einem Photoplethysmographiesensor, einem Elektromyographiesensor, einem elektrische-Feld-Plethysmographiesensor, einem Sensor für die elektrische Hautleitfähigkeit und einem Trägheitsmesseinheit-Sensor.

9. Vorrichtung nach einem der Ansprüche 1 bis 8, wobei das Energiequellenmodul (8), wenn es elektrisch mit dem wiederverwendbaren Modul (1) verbunden ist, die Energieverwaltungseinheit (3) mit elektrischer Energie versorgt.

10. Vorrichtung nach einem der Ansprüche 1 bis 9, wobei das Sensormodul (10), wenn es elektrisch mit dem wiederverwendbaren Modul (1) verbunden ist, elektrische Energie von der Energieverwaltungseinheit (3) empfängt.

11. Vorrichtung nach einem der Ansprüche 2 bis 10, wobei die elektrischen Verbindungsmittel des wiederverwendbaren Moduls (1) zum Verbinden des Sensormoduls (10) mit dem wiederverwendbaren Modul (1) konfiguriert sind, um Sensordaten von dem Sensormodul (10) an die Steuereinheit (2) zu übertragen.

12. Vorrichtung nach einem der Ansprüche 1 bis 11, wobei die Steuereinheit (2) konfiguriert ist, um die Sensordaten von dem Sensormodul (10) zu empfangen, die empfangenen Sensordaten zu verarbeiten und mindestens einen Stimulationsparameter der elektrischen Stimulationssignale basierend auf den verarbeiteten Sensordaten anzupassen.

13. Vorrichtung nach einem der Ansprüche 1 bis 12, wobei das wiederverwendbare Modul (1) eine Signalerfassungsschaltung, die elektrisch mit den elektrischen Verbindungsmitteln zum Verbinden des Elektrodenmoduls (9) mit dem wiederverwendbaren Modul (1) verbunden ist, wobei die Signalerfassungsschaltung konfiguriert ist, um elektrische Impedanz- und Potenzialsignale von dem unter den Elektroden liegenden Gewebe zu messen und aufzuzeichnen, und eine Signalverarbeitungseinheit umfasst, die elektrisch mit der Signalerfassungsschaltung verbunden ist, wobei die Signalverarbeitungseinheit konfiguriert ist, um die gemessenen elektrischen Impedanz- und Potenzialsignale zu analysieren, um physiologische Informationen, wie Herzfrequenz, Herzfrequenzvariabilität und Atemfrequenz, zu extrahieren.

14. Vorrichtung nach Anspruch 13, wobei die Steuereinheit (2) konfiguriert ist, um die physiologischen Informationen von der Signalverarbeitungseinheit zu empfangen und die physiologischen Informationen zu verwenden, um die Stimulationsparameter der elektrischen Stimulationsschaltung (4) basierend auf dem aktuellen physiologischen Zustand des Benutzers und/oder der Reaktion auf die elektrische Stimulation anzupassen.

15. Vorrichtung nach einem der Ansprüche 1 bis 14, umfassend mindestens zwei Sensormodule (10), die unterschiedliche Arten von Sensoren aufweisen.

16. Vorrichtung nach Anspruch 15, wobei ein erstes Sensormodul (10) der mindestens zwei Sensormodule einen Sensor umfasst, der ausgewählt ist aus der Gruppe bestehend aus einem Elektrokardiogrammsensor, einem Elektroenzephalogrammsensor, einem Photoplethysmographiesensor, einem Elektromyographiesensor, einem Sensor für die elektrische Hautleitfähigkeit, einem elektrische-Feld-Plethysmographiesensor und einem Trägheitsmesseinheit-Sensor, und ein zweites Sensormodul (10) der mindestens zwei Sensormodule einen anderen Sensor umfasst, der ausgewählt ist aus der Gruppe bestehend aus einem Elektrokardiogrammsensor, einem Elektroenzephalogrammsensor, einem Photoplethysmographiesensor, einem Elektromyographiesensor, einem Sensor für die elektrische Hautleitfähigkeit, einem elektrische-Feld-Plethysmographiesensor und einem Trägheitsmesseinheit-Sensor.

17. Vorrichtung nach Anspruch 16, wobei das erste Sensormodul (10) und das zweite Sensormodul (10) jeweils ein Verbindungsmittel umfassen, das konfiguriert ist, um mit dem Verbindungsmittel des wiederverwendbaren Moduls zu koppeln, und das Verbindungsmittel des wiederverwendbaren Moduls konfiguriert ist, um entweder das erste Sensormodul oder das zweite Sensormodul aufzunehmen und eine elektrische und mechanische Verbindung damit herzustellen, und wobei die Steuereinheit konfiguriert ist, um zu detektieren, welches von dem ersten Sensormodul oder dem zweiten Sensormodul mit dem Verbindungsmittel des Sensormoduls verbunden ist, und um Daten von dem verbundenen Sensormodul zu empfangen und zu verarbeiten, und optional mindestens einen Stimulationsparameter der elektrischen Stimulationssignale basierend auf den verarbeiteten Daten von dem verbundenen Sensormodul anzupassen.

## Revendications

1. Dispositif de stimulation du nerf auriculaire, comprenant:
un module réutilisable (1) comprenant:
- un circuit de stimulation (4) configuré pour générer des signaux de stimulation électrique,
- une unité de commande (2) configurée pour commander un paramètre de stimulation des signaux de stimulation électrique, tel que l'intensité, la fréquence, la largeur d'impulsion et/ou la durée des signaux de stimulation électrique,
- une unité de communication (5) configurée pour permettre un échange de données entre l'unité de commande (2) et un dispositif externe (6), et
- une unité de gestion d'énergie (3) configurée pour réguler et distribuer l'énergie électrique au circuit de stimulation (4), à l'unité de commande (2) et à l'unité de communication (5),
une pluralité de modules à usage unique comprenant:
- un module de fixation (7) configuré pour être fixé au corps d'un utilisateur,
- un module de source d'énergie (8),
- un module d'électrodes (9) comprenant au moins deux électrodes, et
- facultativement un module de capteur (10),
dans lequel chacun des modules à usage unique (7, 8, 9, 10) et le module réutilisable (1) comprennent des moyens de connexion mécanique pour connecter mécaniquement chacun des modules à usage unique (7, 8, 9, 10) avec le module réutilisable (1) de manière amovible.

2. Dispositif selon la revendication 1, dans lequel le module réutilisable (1), le module d'électrodes (9), le module de source d'énergie (8) et le module de capteur (10) facultatif comprennent des moyens de connexion électrique pour connecter électriquement le module d'électrodes (9), le module de source d'énergie (8) et le module de capteur (10) facultatif, respectivement, avec le module réutilisable (1) de manière amovible.

3. Dispositif selon la revendication 1 ou 2, dans lequel les moyens de connexion mécanique sont configurés pour établir au moins l'une parmi une connexion magnétique, une connexion par friction et une connexion par complémentarité de forme.

4. Dispositif selon la revendication 1, 2 ou 3, dans lequel le circuit de stimulation (4), l'unité de commande (2), l'unité de communication (5) et l'unité de gestion d'énergie (3) sont agencés à l'intérieur d'un boîtier unique du module réutilisable (1).

5. Dispositif selon l'une quelconque des revendications 1 à 4, dans lequel le module d'électrodes (9) comprend des électrodes à aiguille pour délivrer une stimulation percutanée ou des électrodes de surface pour délivrer une stimulation transcutanée.

6. Dispositif selon l'une quelconque des revendications 1 à 5, dans lequel le module de fixation (7) est configuré pour être fixé de manière adhésive au corps d'un utilisateur.

7. Dispositif selon l'une quelconque des revendications 1 à 6, dans lequel au moins deux des modules à usage unique sont combinés dans un boîtier, la combinaison étant sélectionnée dans le groupe constitué de :
a) le module de source d'énergie (8) et le module d'électrodes (9),
b) le module d'électrodes (9) et le module de fixation (7),
c) le module de source d'énergie (8) et le module de fixation (7),
d) le module de source d'énergie (8), le module d'électrodes (9) et le module de fixation (7),
e) le module de capteur (10) et le module de source d'énergie (8), et
f) le module de source d'énergie (8), le module d'électrodes (9), et le module de capteur (10) et facultativement le module de fixation (7).

8. Dispositif selon l'une quelconque des revendications 1 à 7, dans lequel le module de capteur (10) comprend au moins un capteur sélectionné dans le groupe constitué d'un capteur d'électrocardiogramme, d'un capteur d'électroencéphalogramme, d'un capteur de photopléthysmographie, d'un capteur d'électromyographie, d'un capteur de pléthysmographie à champ électrique, d'un capteur de conductance électrique de la peau, et d'un capteur d'unité de mesure inertielle.

9. Dispositif selon l'une quelconque des revendications 1 à 8, dans lequel le module de source d'énergie (8), lorsqu'il est connecté électriquement au module réutilisable (1), fournit de l'énergie électrique à l'unité de gestion d'énergie (3).

10. Dispositif selon l'une quelconque des revendications 1 à 9, dans lequel le module de capteur (10), lorsqu'il est connecté électriquement au module réutilisable (1), reçoit de l'énergie électrique de l'unité de gestion d'énergie (3).

11. Dispositif selon l'une quelconque des revendications 2 à 10, dans lequel les moyens de connexion électrique du module réutilisable (1) pour connecter le module de capteur (10) au module réutilisable (1) sont configurés pour transmettre des données de capteur du module de capteur (10) à l'unité de commande (2).

12. Dispositif selon l'une quelconque des revendications 1 à 11, dans lequel l'unité de commande (2) est configurée pour recevoir les données de capteur du module de capteur (10), traiter les données de capteur reçues, et ajuster au moins un paramètre de stimulation des signaux de stimulation électrique sur la base des données de capteur traitées.

13. Dispositif selon l'une quelconque des revendications 1 à 12, dans lequel le module réutilisable (1) comprend un circuit d'acquisition de signal connecté électriquement aux moyens de connexion électrique pour connecter le module d'électrodes (9) au module réutilisable (1), le circuit d'acquisition de signal étant configuré pour mesurer et enregistrer des signaux d'impédance et de potentiel électriques provenant du tissu sous-jacent aux électrodes, et une unité de traitement de signal connectée électriquement au circuit d'acquisition de signal, l'unité de traitement de signal étant configurée pour analyser les signaux d'impédance et de potentiel électriques mesurés pour extraire des informations physiologiques, telles que la fréquence cardiaque, la variabilité de la fréquence cardiaque et la fréquence respiratoire.

14. Dispositif selon la revendication 13, dans lequel l'unité de commande (2) est configurée pour recevoir les informations physiologiques de l'unité de traitement de signal et utiliser les informations physiologiques pour adapter les paramètres de stimulation du circuit de stimulation électrique (4) sur la base de l'état physiologique actuel de l'utilisateur et/ou de la réponse à la stimulation électrique.

15. Dispositif selon l'une quelconque des revendications 1 à 14, comprenant au moins deux modules de capteur (10) qui ont différents types de capteurs.

16. Dispositif selon la revendication 15, dans lequel un premier module de capteur (10) des au moins deux modules de capteur comprend un capteur sélectionné dans le groupe constitué d'un capteur d'électrocardiogramme, d'un capteur d'électroencéphalogramme, d'un capteur de photopléthysmographie, d'un capteur d'électromyographie, d'un capteur de conductance électrique de la peau, d'un capteur de pléthysmographie à champ électrique, et d'un capteur d'unité de mesure inertielle, et un second module de capteur (10) des au moins deux modules de capteur comprend un autre capteur sélectionné dans le groupe constitué d'un capteur d'électrocardiogramme, d'un capteur d'électroencéphalogramme, d'un capteur de photopléthysmographie, d'un capteur d'électromyographie, d'un capteur de conductance électrique de la peau, d'un capteur de pléthysmographie à champ électrique, et d'un capteur d'unité de mesure inertielle.

17. Dispositif selon la revendication 16, dans lequel le premier module de capteur (10) et le second module de capteur (10) comprennent chacun un moyen de connexion configuré pour s'accoupler avec le moyen de connexion du module réutilisable, et le moyen de connexion du module réutilisable est configuré pour recevoir et établir une connexion électrique et mécanique avec soit le premier module de capteur, soit le second module de capteur, et dans lequel l'unité de commande est configurée pour détecter lequel du premier module de capteur ou du second module de capteur est connecté au moyen de connexion du module de capteur et pour recevoir et traiter des données provenant du module de capteur connecté, et facultativement ajuster au moins un paramètre de stimulation des signaux de stimulation électrique sur la base des données traitées provenant du module de capteur connecté.
